## Europäisches Patentamt
## European Patent Office
## Office européen des brevets

(11) Veröffentlichungsnummer: **0 547 505 B1**

(12) **EUROPÄISCHE PATENTSCHRIFT**

(45) Veröffentlichungstag der Patentschrift: **19.07.95**

(51) Int. Cl.6: **C07C 209/48**, B01J 23/74

(21) Anmeldenummer: **92121052.2**

(22) Anmeldetag: **10.12.92**

(54) **Verfahren zur Herstellung von Aminen durch katalytische Hydrierung von Nitrilen.**

(30) Priorität: **18.12.91 DE 4141771**

(43) Veröffentlichungstag der Anmeldung:
**23.06.93 Patentblatt 93/25**

(45) Bekanntmachung des Hinweises auf die Patenterteilung:
**19.07.95 Patentblatt 95/29**

(84) Benannte Vertragsstaaten:
**BE DE ES FR GB IT NL SE**

(56) Entgegenhaltungen:
**EP-A- 0 322 049**
**EP-A- 0 340 848**
**EP-A- 0 384 542**
**EP-A- 0 398 668**
**EP-A- 0 472 918**

(73) Patentinhaber: **HOECHST AKTIENGESELL-SCHAFT**

**D-65926 Frankfurt (DE)**

(72) Erfinder: **Deckers, Gregor, Dr. Dipl.-Chem.**
**Reeser Strasse 23**
**W-4232 Xanten (DE)**
Erfinder: **Frohning, Dieter, Dr, Dipl.-Chem.**
**Regnitstrasse 50**
**W-4230 Wesel (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Aminen durch katalytische Hydrierung von Nitrilen in Gegenwart eines Nickel enthaltenden Trägerkatalysators. Die katalytische Hydrierung von Nitrilen, insbesondere gesättigten oder ungesättigten Fettsäurenitrilen, führt in Abhängigkeit von den jeweils gewählten Reaktionsbedingungen zu den entsprechenden gesättigten oder ungesättigten primären oder sekundären Aminen.

Die EP 0 384 542 beschreibt ein Verfahren zur Herstellung sekundärer Alkylamine durch katalytische Hydrierung von Alkylnitrilen in Gegenwart eines Nickel enthaltenden Katalysators, der Kupfer und gegebenenfalls Kobalt als Promotor enthält.

Die DE 39 14 875 C2 betrifft ein Verfahren zur katalytischen Hydrierung von aliphatischen Nitrilen zu Aminen unter Verwendung eines Nickelkatalysators auf einem Träger, der Magnesiumoxid und Siliciumdioxid enthält. Der Katalysator weist ein Molverhältnis von Siliciumdioxid zu Nickel zwischen 0,1 und 0,3 und ein Molverhältnis von Magnesium zu Nickel zwischen 0,25 und 0,05 sowie einen durchschnittlichen Porenradius von 1,5 bis 4,0 nm auf.

Allerdings lassen die Verfahren des Standes der Technik trotz vergleichsweise guter Hydrierergebnisse im allgemeinen noch Wünsche offen. Hiervon betroffen sind die Reaktionsbedingungen (Druck, Temperatur), die Aktivität des für die Hydrierung eingesetzten Katalysators, seine Filtrierbarkeit und die Selektivität der Umsetzung. Er-wünscht ist ferner ein Verfahren mit einem breiten Anwendungsbereich, das es gestattet, wahlweise ein gesättigtes primäres Amin, ein gesättigtes sekundäres Amin oder ein ungesättigtes primäres Amin in hoher Ausbeute herzustellen.

Diese Aufgabenstellung wird gelöst durch ein Verfahren zur Herstellung von Aminen durch katalytische Hydrierung von Nitrilen in Gegenwart eines Nickel enthaltenden Trägerkatalysators gegebenenfalls unter Zugabe von Ammoniak bei erhöhter Temperatur und gegebenenfalls erhöhtem Druck, dadurch gekennzeichnet, daß der Trägerkatalysator Mg und Ni in copräzipitierter Form, Mg und Ni in einem Molverhältnis von (0,0075 bis 0,075) : 1 und je Mol Ni 17 bis 60 Gramm eines in Wasser unlöslichen Trägers enthält, die aktive Nickelmetalloberfläche des Trägerkatalysators 110 bis 180 $m^2/g$ Nickel beträgt und 65 bis 97 % der BET-Gesamtoberfläche des Trägerkatalysators von Poren eines Radius $r_p \leq 2,5$ nm gebildet werden.

Ein wesentliches Merkmal des verwendeten Trägerkatalysators ist die enge Verteilung der Porenradien, ausgedrückt durch einen hohen Anteil der BET-Gesamtoberfläche, der auf Poren eines Radius $r_p \leq 2,5$ nm zurückgeht. Unter BET-Gesamtoberfläche wird die durch Adsorption von Stickstoff nach der Brunauer, Emmett und Teller-Methode (BET) ermittelte Oberfläche verstanden.

Die BET-Gesamtoberfläche beträgt 160 bis 450, insbesondere 180 bis 380, bevorzugt 200 bis 350 $m^2/g$ Katalysator. Das Verfahren zur Bestimmung der BET-Gesamtoberfläche nach Brunauer, Emmett und Teller ist in J. Amer. Chem. Soc. 60, (1938) 309 beschrieben.

Durch die vorstehend genannte enge Verteilung der Porenradien mit Schwerpunkt auf einen Bereich $r_p \leq 2,5$ nm erhält der Katalysator besondere Eigenschaften, die eine Erhöhung der Aktivität und eine Verbesserung der Selektivität der dem erfindungsgemäßen Verfahren zugrundeliegenden Umsetzung zur Folge haben.

Ein weiteres Merkmal des Katalysators ist seine Zusammensetzung, ausgedrückt durch das Molverhältnis Mg zu Ni. Der Vollständigkeit halber sei auf die nachfolgende Begriffsbestimmung aufmerksam gemacht. 1 Mol Mg bzw. 1 Mol $Mg^{2+}$ entspricht 24,305 g Mg bzw. 24,305 g $Mg^{2+}$ und 1 Mol Ni bzw. 1 Mol $Ni^{2+}$ entspricht 58,71 g Ni bzw. 58,71 g $Ni^{2+}$. In reduziertem Zustand des Katalysators liegt Magnesium als $Mg^{2+}$ und Nickel überwiegend oder fast vollständig in metallischer Form vor. Unter Molverhältnis ist somit, genauer formuliert, das Verhältnis Mol $Mg^{2+}$ : (Mol Ni + Mol nichtreduziertes $Ni^{2+}$) zu verstehen.

Der Katalysator enthält in reduzierter Form, wie bereits zuvor erwähnt, Mg und Ni in einem Molverhältnis von (0,0075 bis 0,075) : 1. Besonders bewährt hat es sich, ein Molverhältnis Mg : Ni von (0,015 bis 0,060) : 1, insbesondere (0,022 bis 0,055) : 1 zu wählen.

Die Zusammensetzung des neuen Katalysators ist ferner bestimmt durch das Verhältnis der Gewichtsteile des in Wasser unlöslichen Trägers zu Ni, wobei wiederum die Summe aus Mol metallischem Nickel und Mol $Ni^{2+}$ gemeint ist.

Als Träger eignen sich verschiedene, in Wasser unlösliche Materialien. Hierunter fallen Silikate wie Ca-, Mg- und/oder Al-silikate, $Al_2O_3$, $SiO_2$ und/oder Kieselgur. Als besonders brauchbare Träger sind Mg-silikate (insbesondere in Form von Bimsstein), $Al_2O_3$, $SiO_2$ und/oder Kieselgur, insbesondere Bimsstein, $Al_2O_3$, $SiO_2$ und/oder Kieselgur, bevorzugt $SiO_2$ und/oder Kieselgur hervorzuheben. Besonders bewährt hat sich Kieselgur.

Das Trägermaterial soll üblicherweise in feinteiliger Form vorliegen. Seine Teilchen sollen eine Korngröße von 1 bis 30, insbesondere 2 bis 25, bevorzugt 3 bis 20 $\mu$m aufweisen.

Der Katalysator enthält 17,0 bis 60,0, insbesondere 25 bis 50, bevorzugt 30 bis 40 Gramm Träger je Mol Ni. Unter Mol Ni ist - wie bereits zuvor erwähnt - die Summe aus Nickel in reduzierter und nichtreduzierter Form zu verstehen.

Die aktive Nickelmetalloberfläche des Katalysators beträgt 110 bis 180, insbesondere 125 bis 160, bevorzugt 130 bis 150 m$^2$/g Ni. Ihre Bestimmung erfolgt in Anlehnung an eine in Journal of Catalysis 81, (1983) 204 und 96, (1985) 517 näher beschriebene Methode durch Messung der durch Chemisorption bei 20°C aufgenommenen Menge Wasserstoff.

65 bis 97, insbesondere 70 bis 95, bevorzugt 75 bis 95 %, der BET-Gesamtoberfläche werden von Poren eines Radius $r_p$ ≦ 2,5 nm (25 Å) gebildet. Die Bestimmung der Porenradien erfolgt nach einer in S.J. Gregg and K.S.W. Sing, Adsorption Surface Area and Porosity (Academic Press New York-London 1967), Seiten 160 bis 182, näher beschriebenen Methode.

Der Katalysator zeichnet sich ferner dadurch aus, daß 60 bis 95, insbesondere 70 bis 95, bevorzugt 73 bis 90 % der BET-Gesamtoberfläche von Poren mit einem Radius $r_p$ von 1,5 bis 2,5 nm (15 bis 25 Å) gebildet werden.

Poren mit einem Radius $r_p$ von 1,8 bis 2,5 nm (18 bis 25 Å) bilden 35 bis 85, insbesondere 45 bis 76, bevorzugt 50 bis 70 % der BET-Gesamtoberfläche.

Das Verfahren zur Herstellung der verwendeten Katalysatoren geht von einer Nickel- und Magnesiumsalze enthaltenden, wäßrigen Lösung aus. Diese Mischsalzlösung enthält 10 bis 100, insbesondere 20 bis 80, bevorzugt 30 bis 50 g Ni/l. Sie weist Magnesium entsprechend 0,2 bis 15, insbesondere 0,5 bis 12, bevorzugt 1 bis 10 g MgO/l auf.

Die Mischsalzlösung stellt man her, indem man wasserlösliche, anorganische, organische oder komplexe Salze von Nickel und Magnesium in Wasser löst. Gut geeignete Salze sind die Sulfate, Chloride, Acetate, Propionate, Butyrate und Nitrate. Besonders bewährt hat es sich, Nickel und Magnesium in Form ihrer Sulfate, Chloride, Acetate und Nitrate, bevorzugt in Form ihrer Nitrate einzusetzen.

Um einer unerwünschten Hydrolyse vorzubeugen und die Fällung günstig zu beeinflussen, empfiehlt es sich, in der Mischsalzlösung einen Überschuß freier Säure zuzulassen.

Die Mischsalzlösung wird getrennt, aber gleichzeitig mit einer wäßrigen Lösung eines basischen Fällungsmittels einem in Wasser suspendierten Träger zugeführt.

Als Fällungsmittel dient eine wäßrige Lösung einer basischen Verbindung. Besonders geeignet ist eine Na$_2$CO$_3$ und/oder NaHCO$_3$ enthaltende wäßrige Lösung. Das Fällungsmittel soll einen pH-Wert von 7,5 bis 13, insbesondere 8 bis 12, bevorzugt 9 bis 11 aufweisen.

Die wäßrige Lösung enthält 0,1 bis 4,0, insbesondere 0,6 bis 3,0, bevorzugt 1,6 bis 2,4 Äquivalente basische Verbindung/l Lösung. Recht gute Ergebnisse werden mit wäßrigen Lösungen, die 0,3 bis 1,5, insbesondere 0,8 bis 1,2 Mol Alkalicarbonat/l Lösung enthalten, erzielt.

Um eine möglichst vollständige Fällung sicherzustellen und zugleich ein besonders homogenes, aus basischen Nickel- und Magnesiumverbindungen bestehendes Copräzipitat zu erthalten, setzt man die basische Verbindung in geringem Überschuß ein, bezogen auf die zur vollständigen Fällung von Ni und Mg erforderliche Menge basische Verbindung.

Die Fällung wird herbeigefürt, indem man die Mischsalzlösung und das Fällungsmittel getrennt, aber gleichzeitig, kontinuierlich oder diskontinuierlich einem in Wasser suspendierten, für die Herstellung des Katalysators geeigneten Trägermaterial unter Vermischung zuführt.

Als Trägermaterial lassen sich die bereits erwähnten Stoffe, nämlich Silikate des Calciums, des Magnesiums und/oder Aluminiums, Al$_2$O$_3$, SiO$_2$ und/oder Kieselgur, insbesondere Kieselgur verwenden.

Die Fällung des aus basischen Nickel- und Magnesiumverbindungen bestehenden Copräzipitats wird durch vergleichsweise langsame Zugabe der Mischsalzlösung und des Fällungsmittels herbeigeführt. Die Fällungszeit sollte wenigstens 10, insbesondere wenigstens 15, bevorzugt wenigstens 20 Minuten betragen.

Man arbeitet während der Fällung bei einem konstanten pH-Wert innerhalb eines pH-Bereiches von 6 bis 8,5, insbesondere 6,5 bis 7,8. Schwankungen des pH-Wertes sollten möglichst gering gehalten werden.

Die Fällung wird bei Temperaturen oberhalb 80°C, insbesondere in einem Bereich von 90 bis 110, bevorzugt 95 bis 105°C durchgeführt.

Das Copräzipitat enthält Magnesium und Nickel entsprechend einem Molverhältnis von (0,02 bis 0,25) : 1, insbesondere (0,03 bis 0,2) : 1, bevorzugt (0,035 bis 0,1) : 1. Es wird nach Beendigung der Fällung von der Mutterlauge abgetrennt. Dies kann durch Dekantieren und/oder Filtrieren erfolgen.

Anschließend wird das Copräzipitat mit Wasser gewaschen, wobei außer den in Lösung befindlichen Bestandteilen, z.B. Na + und NO$_3^-$, die im Copräzipitat enthaltenen, basischen Magnesiumverbindungen aus dem Copräzipitat herausgelöst werden. Dies hat zur Folge, daß sich das Molverhältnis Magnesium zu Nickel verändert und infolge der Abnahme von Magnesium zu niedrigeren Werten verschiebt. Man wäscht bei relativ hohen Temperaturen von 60 bis 90, insbesondere 65 bis 85, bevorzugt 70 bis 80°C.

Die Dauer des Waschvorganges muß ausreichend bemessen werden. Sie sollte mindestens 60, insbesondere mindestens 80, bevorzugt mindestens 90 Minuten betragen.

Falls gewünscht, kann man die gewaschene Katalysatorzusammensetzung in stückige Form bringen. Zur Formgebung kann man sich bewährter Verfahren, beispielsweise der Strangextrusion bedienen.

Die Trocknung wird bei erhöhten Temperaturen, vorzugsweise bei steigenden Temperaturen stufenweise durchgeführt. Es erweist sich als ausreichend, die Trocknung bei Temperaturen von 50 bis 120, insbesondere 55 bis 100, bevorzugt 60 bis 90°C unter Anwendung üblicher Verfahren, wie Anordnung des Trockengutes im Festbett oder im bewegten Bett, beispielsweise in Wirbelschicht, durchzuführen.

Die Reduktion der Katalysatorzusammensetzung erfolgt mittels Wasserstoff oder Wasserstoff enthaltenden Gasgemischen bei Temperaturen von 260 bis 400, insbesondere 280 bis 360°C.

Ein besonderes Merkmal des erfindungsgemäßen Verfahrens ist sein breiter Anwendungsbereich. Das erfindungsgemäße Verfahren läßt sich nämlich je nach Bedarf sowohl zur Herstellung von gesättigten primären oder sekundären Aminen als auch zur Herstellung von ungesättigten primären Aminen nutzen.

Beabsichtigt man, primäre Amine herzustellen, so führt man die katalytische Hydrierung der Nitrile in Anwesenheit von Ammoniak bei Temperaturen von 80 bis 200, insbesondere 100 bis 180, bevorzugt 120 bis 170°C durch.

Nach einer besonderen Verfahrensvariante stellt man gesättigte primäre Amine durch eine zweistufige katalytische Hydrierung der Nitrile in Anwesenheit von Ammoniak her, wobei zunächst bei Temperaturen von 80 bis 140, insbesondere 110 bis 130 und anschließend bei Temperaturen von 140 bis 180, insbesondere 150 bis 170°C gearbeitet wird.

Ungesättigte primäre Amine lassen sich mit besonders gutem Erfolg herstellen, indem man die katalytische Hydrierung der Nitrile in Anwesenheit von Ammoniak bei Temperaturen von 100 bis 140, insbesondere 110 bis 130°C durchführt.

Zur Herstellung primärer Amine, d. h. sowohl gesättigter als auch ungesättigter primärer Amine führt man die katalytische Hydrierung unter einem Druck von 1,0 bis 6,0, insbesondere 1,5 bis 5,0, bevorzugt 2,0 bis 4,0 MPa durch.

Es empfiehlt sich, die Umsetzung unter Zugabe von Ammoniak durchzuführen. Dadurch wird die Selektivität der Umsetzung verbessert und es werden Spaltungsreaktionen, die beispielsweise zur Freisetzung von Ammoniak führen, zurückgedrängt. Üblicherweise setzt man der Reaktion 2 bis 10 Gew.-% Ammoniak, bezogen auf Nitril, zu.

Die Herstellung sekundärer Amine erfordert andere Bedingungen als die Synthese gesättigter oder ungesättigter primärer Amine. Im allgemeinen liegen die erforderlichen Temperaturen höher. Es hat sich bewährt, die katalytische Hydrierung der Nitrile bei Temperaturen von 160 bis 250, insbesondere 170 bis 240, bevorzugt 180 bis 220°C durchzuführen. Der Druck beträgt 0,1 bis 5,0, insbesondere 0,2 bis 3,0, bevorzugt 0,3 bis 2,0 MPa. Auf eine Zugabe von Ammoniak kann verzichtet werden. Die Herstellung sekundärer Amine läßt sich besonders vorteilhaft gestalten, indem man das während der katalytischen Hydrierung der Nitrile freigesetzte Ammoniak laufend aus der Umsetzung entfernt. Dies erreicht man beispielsweise mittels Durchleiten eines Wasserstoffstromes, mit dessen Hilfe Ammoniak aus dem resultierenden Reaktionsgemisch ausgetrieben wird.

Das erfindungsgemäße Verfahren eignet sich besonders für eine diskontinuierliche Arbeitsweise, es kann jedoch auch kontinuierlich oder halbkontinuierlich durchgeführt werden. Bei diskontinuierlicher Arbeitsweise setzt man den Nickel enthaltenden Trägerkatalysator entsprechend 0,05 bis 2,0, insbesondere 0,08 bis 1,0, bevorzugt 0,1 bis 0,5 Gew.-% Nickel, bezogen auf Nitril, ein.

Als Ausgangsstoffe eignen sich Nitrile mit 6 bis 24 Kohlenstoffatomen, insbesondere geradkettige und/oder verzweigte aliphatische Nitrile mit 8 bis 22 Kohlenstoffatomen, bevorzugt geradkettige und/oder verzweigte aliphatische Nitrile mit 10 bis 22 Kohlenstoffatomen.

Das erfindungsgemäße Verfahren läßt sich mit besonders gutem Erfolg bei der Hydrierung von Nitrilen, die sich von höheren natürlichen Fettsäuren, beispielsweise der Laurinsäure, der Palmitinsäure, der Stearinsäure und der Ölsäure ableiten, insbesondere Mischungen dieser Nitrile oder Nitrile technischer Qualität anwenden. Ein derartiges Material technischer Qualität ist Talgfettsäurenitril, das im wesentlichen aus Nitrilen der Stearinsäure, der Palmitinsäure und Ölsäure besteht.

Die nachstehenden Versuche belegen die Erfindung, ohne sie einzuschränken.

4

## Experimenteller Teil

## ====================

Beispiel 1

Herstellung von ungesättigtem, primärem Talgfettamin

400 g Talgfettsäurenitril (destilliertes Material) werden in einem mit $N_2$ gespülten Rührautoklav (Volumen 1 Liter) vorgelegt. Es wird ein in höhere Fettamine eingehüllter Katalysator verwendet, der, bezogen auf Katalysatorgesamtmasse, etwa 64 Gew.-% Fettamine und 19,5 Gew.-% Ni, je Mol Ni 36,34 g Träger (Kieselgur) und Mg:Ni in einem Molverhältnis 0,04 : 1 enthält. Der Katalysator besitzt eine aktive Nickelmetalloberfläche von 120 $m^2$/g Nickel und eine BET-Gesamtoberfläche von 220 $m^2$/g Katalysator (Katalysator gerechnet ohne das einhüllende Fettamin), wobei 80 % der BET-Gesamtoberfläche von Poren eines Radius $r_p \leq$ 2,5 nm (25 Å) gebildet werden (Filtrationszeit des Katalysators: 2,33 min).

4,1 g dieses Katalysators sowie 24 g $NH_3$ werden zugegeben. Anschließend wird Wasserstoff zunächst bis zu einem Druck von 2,1 MPa aufgepreßt und unter Rühren auf die vorgegebene Reaktionstemperatur (125 ° C) erhitzt. Der Reaktionsdruck (3,1 MPa) wird durch kontinuierliche Zugabe von Wasserstoff eingestellt und aufrechterhalten.

Vergleichsversuch 1

Herstellung von ungesättigtem Talgfettamin

Wie in Beispiel 1 angegeben, werden 400 g Talgfettnitril (destilliertes Material) in einem mit $N_2$-gespülten Rührautoklaven (Volumen 1 Liter) vorgelegt. 10 g eines Raney-Nickel-Katalysators, der etwa 60 Gew.-% Nickel, bezogen auf Gesamtmasse Katalysator, umhüllt von Distearylamin enthält, sowie 24 g $NH_3$ werden vorgelegt. Anschließend wird Wasserstoff zunächst bis zu einem Druck von 2,1 MPa aufgepreßt und unter Rühren auf die vorgegebene Reaktionstemperatur (125 ° C) erhitzt. Der Reaktionsdruck (3,1 MPa) wird durch kontinuierliche Zugabe von Wasserstoff eingestellt und aufrechterhalten.

Vergleichsversuch 2

Herstellung von ungesättigtem Talgfettamin

Wie in Beispiel 1 angegeben, werden 400 g Talgfettsäurenitril (destilliertes Material) in einem mit $N_2$ gespülten Autoklav (Volumen 1 Liter) vorgelegt. 4,35 g eines handelsüblichen, für die Hydrierung von Nitrilen empfohlenen Trägerkatalysators, der 20,7 Gew.-% Ni, 0,1 Gew.-% MgO, etwa 3,2 Gew.-% $SiO_2$ und etwa 3,5 Gew.-% $Al_2O_3$ umhüllt von Distearylamin enthält und eine Filtrationszeit von 6,74 min aufweist, sowie 24 g $NH_3$ werden zugegeben. Anschließend wird Wasserstoff zunächst bis zu einem Druck von 2,1 MPa aufgepreßt und unter Rühren auf die vorgegebene Reaktionstemperatur (125 ° C) erhitzt. Der Reaktionsdruck (3,1 MPa) wird durch kontinuierliche Zugabe von Wasserstoff eingestellt und aufrechterhalten.

Die Reaktionsbedingungen und die Zusammensetzung der erhaltenen Reaktionsprodukte sind in der nachfolgenden Tabelle 1 zusammengefaßt.

## Tabelle 1

|  | Beispiel 1 | Vergleichsversuch 1 | Vergleichsversuch 2 |
|---|---|---|---|
| **Reaktionsbedingungen** | | | |
| Nickel bezogen auf Einsatzstoff* | 0,2 Gew.-% | 1,5 Gew.-% | 0,225 Gew.-% |
| Temperatur | 125°C | 125°C | 125°C |
| Druck | 3,1 MPa | 3,1 MPa | 3,1 MPa |
| $NH_3$ bezogen auf Einsatzstoff | 6 Gew.-% | 6 Gew.-% | 6 Gew.-% |
| Reaktionszeit insgesamt** | 270 min | 270 min | 270 min |
| $H_2$-Aufnahme | 170 min | 240 min | 210 min |
| **Zusammensetzung des Reaktionsproduktes (in Gew.-%)** | | | |
| Primäre Amine | 96,5 | 96,4 | 95,8 |
| Sekundäre Amine | 1,9 | 2,1 | 2,5 |
| Tertiäre Amine | 0,9 | 0,9 | 0,8 |
| Nicht-Amine | 0,7 | 0,6 | 0,9 |
| Jodzahl (g $J_2$/100 g) | 52 | 31 | 51 |

* Einsatzstoff: Talgfettsäurenitril mit einem Nitrilgehalt von > 99,5 Gew.-% (gaschromatografisch ermittelt), Jodzahl: 56,9 (g $J_2$/100 g), 0,01 Gew.-% Wasser, 0,028 Gew.-% Amidstickstoff neben Spuren von Na, S, Cl und P.

** einschließlich 40 min Aufheizzeit

Analytische Bestimmung des Reaktionsproduktes

Das hydrierte Produkt wird hinsichtlich Jodzahl, Amin-Gehalt und Nicht-Amin-Gehalt untersucht.

Der Gesamtamin-Gehalt wird durch Titration mittels in Isopropanol gelöster HCl bestimmt. Zur Bestimmung der sekundären und tertiären Amine werden zunächst die primären Amine mit Salicylaldehyd in die entsprechenden Azomethine (Schiffsche Base) überführt und die verbleibenden sekundären und tertiären Amine anschließend durch Titration mittels in Isopropanol gelöster HCl ermittelt. Zur Bestimmung der tertiären Amine werden zunächst die primären und sekundären Amine mit Essigsäureanhydrid in die entsprechenden Derivate umgewandelt und die verbleibenden tertiären Amine durch Titration mittels in Essigsäure gelöster $HClO_4$ ermittelt.

Die analytische Erfassung des Nicht-Amin-Gehaltes erfolgt durch gaschromatografische Analyse des Reaktionsproduktes, aus dem zuvor mittels eines sauren Ionenaustauschers die gesamten Amine entfernt wurden. Hierbei handelt es sich um eine Bestimmung in Anlehnung an ASTM D 2082-82. Bei den Nicht-Aminen handelt es sich beispielsweise um Alkohole, Carbonsäuren, Amide und Ester, nicht jedoch um nichtumgesetztes Nitril (Einsatzstoff), wie eine infrarotspektroskopische Untersuchung belegt (ausgenommen die Vergleichsversuche 5, 6 und 7).

Beispiel 2

Herstellung von Dodecylamin

200 g Dodecylnitril werden in einem mit $N_2$ gespülten Rührautoklav (Volumen 1 Liter) vorgelegt. 3,08 g des in Beispiel 1 beschriebenen Katalysators sowie 20 g $NH_3$ werden zugegeben. Anschließend wird Wasserstoff zunächst bis zu einem Druck von 1,6 MPa aufgepreßt und unter Rühren auf die vorgegebene Reaktionstemperatur (140 °C) erhitzt. Der Reaktionsdruck (2,6 MPa) wird durch kontinuierliche Zugabe von Wasserstoff aufrechterhalten. Die Umsetzung ist nach 60 Minuten abgeschlossen.

Die Reaktion liefert primäres Dodecylamin in einer Ausbeute > 96 % der Theorie. Der Nicht-Amin-Gehalt beträgt < 1 Gew.-%.

Beispiel 3

Herstellung gesättigter, primärer Amine

400 g Talgfettsäurenitril (destilliertes Material wie in Beispiel 1 angegeben) werden in einem mit $N_2$ gespülten Autoklav (Volumen 1 Liter) vorgelegt. 6,15 g des in Beispiel 1 beschriebenen Katalysators (entsprechend 0,3 Gew.-% Ni bezogen auf Talgfettsäurenitril) sowie 24 g $NH_3$ werden zugegeben. Anschließend wird Wasserstoff aufgepreßt und unter Rühren auf die vorgegebene Reaktionstemperatur erhitzt. Die Umsetzung erfolgt in zwei Stufen:

1. Stufe:

| | |
|---|---|
| Temperatur | 130 °C |
| Druck | 3,1 MPa |
| Reaktionszeit | 100 min |

Im Anschluß an die 1. Stufe wird der Autoklav entspannt und es wird mit Wasserstoff gespült, wodurch noch vorhandenes $NH_3$ entfernt wird. Nachfolgend wird erneut Wasserstoff aufgepreßt und unter Rühren auf die vorgegebene Reaktionstemperatur der 2. Stufe erhitzt.

2. Stufe:

| Temperatur | 150 °C |
| Druck | 3,1 MPa |
| Reaktionszeit | ca. 140 min |

Die zweistufige Umsetzung des Talgfettsäurenitrils (Jodzahl 56,9 (g $J_2$/100 g)) führt zu einem Reaktionsprodukt mit einem Gehalt an primären Aminen $\geq$ 93 Gew.-% und einem Nicht-Amin-Gehalt von $\leq$ 0,7 Gew.-%. Die Jodzahl beträgt < 5 (g $J_2$/100 g).

Beispiel 4

Herstellung von sekundärem gesättigtem Talgfettamin (Distearylamin)

500 g Talgfettsäurenitril (destilliertes Material wie in Beispiel 1 angegeben) werden in einem mit $N_2$ gespülten Rührautoklav (Volumen 1 Liter) vorgelegt. 4,76 g eines in höhere Fettamine eingehüllten Katalysators werden zugegeben.

Der Katalysator enthält, bezogen auf Katalysatorgesamtmasse, etwa 63 Gew.-% Fettamine und 21 Gew.-% Ni, je Mol Ni 36,3 Träger (Kieselgur) und Mg : Ni in einem Molverhältnis 0,055 : 1. Er besitzt eine aktive Nickelmetalloberfläche von 141 $m^2$/g Nickel und eine BET-Gesamtoberfläche von 310 $m^2$/g Katalysator (Katalysator gerechnet ohne das einhüllende Fettamin), wobei 83 % der BET-Gesamtoberfläche von Poren eines Radius $r_p \leq$ 2,5 nm (25 Å) gebildet werden.

Der für die Umsetzung benötigte Wasserstoff wird über ein mit einer Verteilervorrichtung versehenes Tauchrohr unter Rühren durch die den Katalysator enthaltende Suspension kontinuierlich und im Überschuß hindurchgeleitet. Zugleich wird das Abgas, das nichtumgesetzten Wasserstoff und durch die Reaktion gebildetes Ammoniak enthält, abgeleitet. Die Zugabe von Wasserstoff und das Ableiten von Abgas werden so eingestellt, daß während des Aufheizens und während der nachfolgenden Reaktion ein Druck von 0,3 MPa aufrechterhalten wird.

Man heizt binnen 45 Minuten auf eine Temperatur von 200 °C auf und setzt die Reaktion über einen Zeitraum von 4 Stunden fort.

Vergleichsversuch 3

Herstellung von sekundärem gesättigtem Talgfettamin (Distearylamin)

500 g Talgfettsäurenitril (destilliertes Material wie in Beispiel 1 angegeben) werden in einem mit $N_2$ gespülten Rührautoklav (Volumen 1 Liter) vorgelegt. 5,56 g eines handelsüblichen für die Umsetzung von Nitrilen zu sekundären Fettaminen empfohlenen Katalysators, der nach Angaben des Herstellers 18 Gew.-% Nickel, etwa 2 Gew.-% Kupfer, etwa 7 Gew.-% Träger und daneben überwiegend sekundäres Amin als Schutzumhüllung enthält, werden zugegeben.

Anschließend wird wie in Beispiel 4 angegeben gearbeitet. Im Reaktionsprodukt sind allerdings, wie die Jodzahl in Tabelle 2 ausweist, noch erhebliche Mengen ungesättigter sekundärer Amine enthalten.

Vergleichsversuch 4

Herstellung von sekundärem gesättigtem Talgfettamin (Distearylamin)

Es wird wie in Vergleichsversuch 3 angegeben gearbeitet, jedoch 27,8 g des im Vergleichsversuch 1 beschriebenen Katalysators eingesetzt.

Die Reaktionsbedingungen und die Zusammensetzung der erhaltenen Reaktionsprodukte sind in der nachfolgenden Tabelle 2 zusammengefaßt.

## Tabelle 2

### Reaktionsbedingungen

| | Beispiel 4 | Vergleichsversuch 3 | Vergleichsversuch 4 |
|---|---|---|---|
| Nickel bezogen auf Einsatzstoff* | 0,2 Gew.-% | 0,2 Gew.-% | 1,0 Gew.-% |
| Temperatur | 200°C | 200°C | 200°C |
| Druck | 0,3 MPa | 0,3 MPa | 0,3 MPa |
| Reaktionszeit insgesamt** | 285 min | 285 min | 285 min |

### Zusammensetzung des Reaktionsproduktes (in Gew.-%)

| | Beispiel 4 | Vergleichsversuch 3 | Vergleichsversuch 4 |
|---|---|---|---|
| Primäre Amine | 0,7 | 6,1 | 1,4 |
| Sekundäre Amine | 94,0 | 91,7 | 91,8 |
| Tertiäre Amine | 4,6 | 1,7 | 4,6 |
| Nicht-Amine | 0,7 | 0,5 | 2,2 |
| Jodzahl (g $J_2$/100 g) | 0,7 | 27,0 | 3,9 |

* Einsatzstoff: Talgfettsäurenitril wie in Tabelle 1 angegeben

** einschließlich 45 min Aufheizzeit

Beispiel 5

Herstellung von ungesättigtem, primärem Talgfettamin

400 g Talgfettsäurenitril (destilliertes Material, Nitrilgehalt gaschromatografisch ermittelt: > 99,5 Gew.-%, Jodzahl: 51,7 (g $J_2$/100 g), 0,01 Gew.-% Wasser, 0,02 Gew.-% Amidstickstoff neben Spuren von Na, S, Cl

9

EP 0 547 505 B1

und P) werden mit 3,81 g des in Beispiel 4 beschriebenen Katalysators (entsprechend 0,2 Gew.-% Ni bezogen auf Talgfettsäurenitril) sowie 24 g $NH_3$ zugegeben.

Anschließend wird wie in Beispiel 1 angegeben gearbeitet. Nach 160 min ist die Wasserstoffaufnahme beendet. Das Reaktionsprodukt weist die nachfolgende Zusammensetzung (in Gew.-%) auf:

| | |
|---|---|
| Primäre Amine | 96,6 |
| Sekundäre Amine | 2,1 |
| Tertiäre Amine | 0,8 |
| Nicht-Amine | 0,5 |
| Jodzahl (g $J_2$/100 g) | 49 |

Beispiel 6

Herstellung von ungesättigtem, primären Talgfettamin

Es wird ein strangextrudierter Katalysator, der nicht von Fettamin umhüllt ist, verwendet. Der Katalysator enthält, bezogen auf Katalysatorgesamtmasse, 59,7 Gew.-% Ni, je Mol Ni 33,2 g Träger (Kieselgur), und Mg : Ni in einem Molverhältnis 0,03 : 1. Er besitzt eine aktive Nickelmetalloberfläche von 129 $m^2$/g Nickel und eine BET-Gesamtoberfläche von 286 $m^2$/g Katalysator, wobei 74,4 % der BET-Gesamtoberfläche von Poren eines Radius $r_p \leq$ 2,5 nm (25 Å), 73 % der BET-Gesamtoberfläche von Poren eines Radius $r_p$ = 1,5 bis 2,5 nm (15 bis 25 Å) und 64 % der BET-Gesamtoberfläche von Poren eines Radius $r_p$ = 1,8 bis 2,5 nm (18 bis 25 Å) gebildet werden.

1,34 g dieses strangextrudierten Katalysators entsprechend 0,2 Gew.-% Ni, bezogen auf Talgfettsäurenitril, werden in einem mit $N_2$ gespülten Autoklaven (Volumen 1 Liter) vorgelegt und mit Hilfe eines Pistills durch Zerdrücken verkleinert.

Anschließend werden 400 g Talgfettsäurenitril (destilliertes Material, Nitrilgehalt gaschromatografisch ermittelt: > 99,5 Gew.-%, Jodzahl 55 (g $J_2$/100 g) und 24 $NH_3$ zugesetzt. Anschließend wird, wie in Beispiel 1 angegeben, gearbeitet. Die Wasserstoffaufnahme ist nach 210 min beendet. Dieser im Vergleich zu Beispiel 1 höhere Wert ist darauf zurückzuführen, daß der Katalysator nicht so feinteilig, wie der in Beispiel 1 eingesetzte Katalysator, vorliegt.

Das Reaktionsprodukt weist die nachfolgende Zusammensetzung (in Gew.-%) auf:

| | |
|---|---|
| Primäre Amine | 95,9 |
| Sekundäre Amine | 2,6 |
| Tertiäre Amine | 0,6 |
| Nicht-Amine | 0,9 |
| Jodzahl (g $J_2$/100 g) | 47,5 |

Beispiel 7

Herstellung von ungesättigtem, primärem Oleylamin

500 g Oleylnitril (destilliertes Material, Nitrilgehalt gaschromatografisch ermittelt: ca. 99,5 %, Jodzahl 92,8 (g $J_2$/100 g), 0,02 Gew.-% Amidstickstoff) werden in einem mit N2 gespülten Rührautoklav (Volumen 1 Liter) vorgelegt. Es wird ein in höhere Fettamine eingehüllter Katalysator verwendet, der bezogen auf Katalysatorgesamtmasse etwa 63 Gew.-% Fettamine und 21 Gew.-% Ni, je Mol Ni etwa 36 g Träger (Kieselgur) und Mg : Ni in einem Molverhältnis 0,053 : 1 enthält. Der Katalysator besitzt eine aktive Nickelmetalloberfläche von 125 $m^2$/g Nickel und eine BET-Gesamtoberfläche von 273 $m^2$/g Katalysator (Katalysator gerechnet ohne das umhüllende Fettamin), wobei 71 % der BET-Gesamtoberfläche von Poren eines Radius $r_p \leq$ 2,5 nm (25 Å) gebildet werden.

4,76 g dieses Katalysators (entsprechend 0,2 Gew.-% Ni, bezogen auf Oleylnitril) sowie 30 g $NH_3$ werden zugegeben.

Anschließend wird, wie in Beispiel 1 angegeben, gearbeitet, allerdings wird innerhalb von 30 min auf die Reaktionstemperatur erhitzt.

Die Wasserstoffaufnahme ist nach 320 min beendet.

Vergleichsversuch 5

Herstellung von ungesättigtem, primärem Oleylamin

Es wird, wie in Beispiel 7 beschrieben, gearbeitet, mit dem Unterschieb, daß 5 g des in Vergleichsversuch 2 beschriebenen Katalysators eingesetzt werden.

Die Reaktionsbedingungen und die Zusammensetzung der nach Beispiel 7 und Vergleichsversuch 5 erhaltenen Reaktionsprodukte sind in der nachfolgenden Tabelle 3 zusammengestellt.

## Tabelle 3

| | Beispiel 7 | Vergleichsversuch 5 |
|---|---|---|
| **Reaktionsbedingungen** | | |
| Nickel bezogen auf Einsatzstoff | 0,2 Gew.-% | 0,2 Gew.-% |
| Temperatur | 125°C | 125°C |
| Druck | 3,1 MPa | 3,1 MPa |
| $H_2$-Aufnahme | 320 min | 335 min |
| Reaktionszeit insgesamt* | 365 min | 365 min |
| **Zusammensetzung des Reaktionsproduktes (in Gew.-%)** | | |
| Primäre Amine | 96,6 | 43,5 |
| Sekundäre Amine | 1,7 | 0,3 |
| Tertiäre Amine | 0,6 | 0,1 |
| Nicht-Amine | 1,1 | 56,4** |
| Jodzahl (g $J_2$/100 g) | 88,5 | 89,7 |

* einschließlich 30 min Aufheizzeit

** überwiegend nicht umgesetzter Einsatzstoff

## Beispiel 8

Herstellung von ungesättigtem, primärem Talgfettamin

400 g Talgfettsäurenitril (destilliertes Material wie in Beispiel 6 angegeben) werden in einem mit $N_2$ gespülten Rührautoklav (Volumen 1 Liter) vorgelegt.

3,81 g des in Beispiel 7 beschriebenen Katalysators sowie 24 g $NH_3$ werden zugegeben. Anschließend wird, wie in Beispiel 1 angegeben, gearbeitet, allerdings wird innerhalb von 30 min auf die Reaktionstempe-

12

ratur erhitzt.

Die Reaktionszeit beträgt 165 min.

Vergleichsversuch 6

Herstellung von ungesättigtem, primärem Talgfettamin

Es wird, wie in Beispiel 8 angegeben, gearbeitet, mit dem Unterschied, daß 1,33 des in Vergleichsversuch 1 beschriebenen Katalysators (entsprechend 0,2 Gew.-% Ni bezogen auf Talgfettsäurenitril) eingesetzt werden.

Die Reaktionszeit beträgt 165 min.

Vergleichsversuch 7

Herstellung von ungesättigtem, primären Talgfettamin

Es wird, wie in Beispiel 8 angegeben, gearbeitet, mit dem Unterschied, daß 4,0 g des in Vergleichsversuch 2 beschriebenen Katalysators (entsprechend 0,2 Gew.-% Ni bezogen auf Talgfettsäurenitril) eingesetzt werden.

Die Reaktionszeit beträgt 165 min.

Die Reaktionsbedingungen und die Zusammensetzung der nach Beispiel 8 und den Vergleichsversuchen 6 und 7 erhaltenen Reaktionsprodukte sind in der nachfolgenden Tabelle 4 zusammengestellt.

Tabelle 4

|  | Beispiel 8 | Vergleichsversuch 6 | Vergleichsversuch 7 |
|---|---|---|---|
| Reaktionsbedingungen |  |  |  |
| Nickel bezogen auf Einsatzstoff | 0,2 Gew.-% | 0,2 Gew.-% | 0,2 Gew.-% |
| Temperatur | 125°C | 125°C | 125°C |
| Druck | 3,1 MPa | 3,1 MPa | 3,1 MPa |
| Beginn der $H_2$-Aufnahme | 107°C | 104°C | 124°C |
| Reaktionszeit insgesamt* | 195 min | 195 min | 195 min |
| Zusammensetzung des Reaktionsproduktes (in Gew.-%) |  |  |  |
| Primäre Amine | 94,4 | 76,8 | 21,4 |
| Sekundäre Amine | 2,4 | 1,5 | 1,8 |
| Tertiäre Amine | 0,8 | 0,8 | 0,6 |
| Nicht-Amine | 1,4 | etwa 20** | etwa 75** |
| Jodzahl (g $J_2$/100 g) | 51,5 | 52,6 | 53,6 |

\* einschließlich 30 min Aufheizzeit

\** überwiegend nichtumgesetzter Einsatzstoff

**Patentansprüche**

1. Verfahren zur Herstellung von Aminen durch katalytische Hydrierung von Nitrilen in Gegenwart eines Nickel enthaltenden Trägerkatalysators gegebenenfalls unter Zugabe von Armmmoniak bei erhöhter

Temperatur und gegebenenfalls erhöhtem Druck, dadurch gekennzeichnet, daß der Trägerkatalysator Mg und Ni in copräzipitierter Form, Mg und Ni in einem Molverhältnis von (0,0075 bis 0,075):1 und je Mol Ni 17 bis 60,0 Gramm eines in Wasser unlöslichen Trägers enthält, die aktive Nickelmetalloberfläche des Trägerkatalysators 110 bis 180 $m^2$/g Ni beträgt und 65 bis 97 % der BET-Gesamtoberfläche des Trägerkatalysators von Poren eines Radius $r_p \leq 2,5$ nm gebildet werden.

2. Verfahren nach Anspruch 1, dadurch gekennzeichnet, daß die BET-Gesamtoberfläche des Trägerkatalysators 160 bis 450, insbesondere 180 bis 380, bevorzugt 200 bis 350 $m^2$/g Katalysator beträgt.

3. Verfahren nach Anspruch 1 oder 2, dadurch gekennzeichnet, daß der Trägerkatalysator Mg und Ni in einem Molverhältnis von (0,015 bis 0,06) : 1, insbesondere (0,022 bis 0,055) : 1 enthält.

4. Verfahren nach einem oder mehreren der Ansprüche 1 bis 3, dadurch gekennzeichnet, daß der Trägerkatalysator Ca-, Mg-, Al-silikat, $Al_2O_3$, $SiO_2$ und/oder Kieselgur, insbesondere $Al_2O_3$, $SiO_2$ und/oder Kieselgur, bevorzugt $SiO_2$ und/oder Kieselgur, besonders bevorzugt Kieselgur als Träger enthält.

5. Verfahren nach einem oder mehreren der Ansprüche 1 bis 4 dadurch gekennzeichnet, daß zur Herstellung primärer Amine die katalytische Hydrierung der Nitrible in Anwesenheit von Ammoniak bei Temperaturen von 80 bis 200, insbesondere 100 bis 180, bevorzugt 120 bis 170°C durchgeführt wird.

6. Verfahren nach Anspruch 5, dadurch gekennzeichnet, daß die katalytische Hydrierung unter einem Druck von 1,0 bis 6,0, insbesondere 1,5 bis 5,0, bevorzugt 2,0 bis 4,0 MPa durchgeführt wird.

7. Verfahren nach einem oder mehreren der Ansprüche 1 bis 4, dadurch gekennzeichnet, daß zur Herstellung sekundärer Amine die katalytische Hydrierung der Nitrile bei Temperaturen von 160 bis 250, insbesondere 170 bis 240, bevorzugt 180 bis 220°C durchgeführt wird.

8. Verfahren nach Anspruch 7, dadurch gekennzeichnet, daß die katalytische Hydrierung unter einem Druck von 0,1 bis 5,0, insbesondere 0,2 bis 3,0, bevorzugt 0,3 bis 2,0 MPa durchgeführt und freigesetztes Ammoniak aus der Umsetzung entfernt wird.

9. Verfahren nach einem oder mehreren der Ansprüche 1 bis 8, dadurch gekennzeichnet, daß der Nickel enthaltende Trägerkatalysator entsprechend 0,05 bis 2,0, insbesondere 0,08 bis 1,0 bevorzugt 0,1 bis 0,5 Gew.-% Nickel, bezogen auf Nitril, eingesetzt wird.

10. Verfahren nach einem oder mehreren der Ansprüche 1 bis 9, dadurch gekennzeichnet, daß Nitrile mit 6 bis 24 Kohlenstoffatomen, insbesondere geradkettige und/oder verzweigte aliphatische Nitrile mit 8 bis 22 Kohlenstoffatomen, bevorzugt geradkettige und/oder verzweigte aliphatische Nitrile mit 10 bis 22 Kohlenstoffatomen eingesetzt werden.

**Claims**

1. Process for preparing amines by catalytic hydrogenation of nitriles in the presence of a nickel-containing support catalyst, with the optional addition of ammonia, at elevated temperature and optionally at elevated pressure, characterised in that the support catalyst contains Mg and Ni in coprecipitated form, Mg and Ni in a molar ratio of (0.0075 to 0.075):1 and 17 to 60.0 g of a water-insoluble support per mole of nickel, the active nickel metal surface of the support catalyst is 110 to 180 $m^2$/g of nickel and 65 to 97% of the BET total surface area of the support catalyst is formed by pores having a radius $r_p \leq 2.5$ nm.

2. Process according to claim 1, characterised in that the BET total surface area of the support catalyst is 160 to 450, in particular 180 to 380, preferably 200 to 350 $m^2$/g of catalyst.

3. Process according to claim 1 or 2, characterised in that the support catalyst contains Mg and Ni in a molar ratio of (0.015 to 0.06):1, in particular (0.022 to 0.055):1.

**4.** Process according to one or more of the claims 1 to 3, characterised in that the support catalyst contains calcium silicate, magnesium silicate, aluminium silicate, $Al_2O_3$, $SiO_2$ and/or kieselguhr, in particular $Al_2O_3$, $SiO_2$ and/or kieselguhr, preferably $SiO_2$ and/or kieselguhr, particularly preferably kieselguhr as a support.

**5.** Process according to one or more of the claims 1 to 4, characterised in that, in order to prepare primary amines, the catalytic hydrogenation of the nitriles is performed in the presence of ammonia at temperatures of 80 to 200, in particular 100 to 180, preferably 120 to 170 ° C.

**6.** Process according to claim 5, characterised in that the catalytic hydrogenation is performed at a pressure of 1.0 to 6.0, in particular 1.5 to 5.0, preferably 2.0 to 4.0 MPa.

**7.** Process according to one or more of the claims 1 to 4, characterised in that, in order to prepare secondary amines, the catalytic hydrogenation of the nitriles is performed at temperatures of 160 to 250, in particular 170 to 240, preferably 180 to 220 ° C.

**8.** Process according to claim 7, characterised in that the catalytic hydrogenation is performed at a pressure of 0.1 to 5.0, in particular 0.2 to 3.0, preferably 0.3 to 2.0 MPa and the ammonia released from the reaction is removed.

**9.** Process according to one or more of the claims 1 to 8, characterised in that the nickel-containing support catalyst is used in an amount corresponding to 0.05 to 2.0, in particular 0.08 to 1.0, preferably 0.1 to 0.5 wt % of nickel, related to nitrile.

**10.** Process according to one or more of the claims 1 to 9, characterised in that nitriles having 6 to 24 carbon atoms, in particular straight-chain and/or branched aliphatic nitriles having 8 to 22 carbon atoms, preferably straight-chain and/or branched aliphatic nitriles having 10 to 22 carbon atoms are used.

**Revendications**

**1.** Procédé de préparation d'amines par hydrogénation catalytique de nitriles en présence d'un catalyseur sur support contenant du nickel, éventuellement avec adjonction d'ammoniac, à température élevée et le cas échéant sous pression, caractérisé en ce que le catalyseur sur support contient Mg et Ni à l'état coprécipité, à un rapport molaire Mg/Ni de 0,0075 à 0,075:1, et 17 à 60,0 g d'un support insoluble dans l'eau par mole de Ni, la surface active de nickel métallique de ce catalyseur sur support est de 110 à 180 $m^2$/g de Ni, et 65 à 97% de la surface totale BET du catalyseur sur support sont constitués de pores de rayon $r_p \leq 2,5$ nm.

**2.** Procédé selon revendication 1, caractérisé en ce que la surface totale BET du catalyseur sur support est de 160 à 450 plus spécialement de 180 à 380 et de préférence de 200 à 350 $m^2$/g de catalyseur.

**3.** Procédé selon revendication 1 ou 2, caractérisé en ce que le catalyseur sur support contient Mg et Ni à un rapport molaire de 0,015 à 0,06: 1, plus spécialement de 0,022 à 0,055:1.

**4.** Procédé selon une ou plusieurs des revendications 1 à 3, caractériséen ce que le catalyseur sur support contient en tant que support un silicate de Ca, de Mg, de Al, $Al_2O_3$, $SiO_2$ et/ou du kieselguhr, plus spécialement $Al_2O_3$, $SiO_2$ et/ou du kieselguhr, de préférence $SiO_2$ et/ou du kieselguhr, et surtout du kieselguhr.

**5.** Procédé selon une plusieurs des revendications 1 à 4, caractérisé en ce que, pour la préparation des amines primaires, on procéde à l'hydrogénation catalytique des nitriles en présence d'ammoniac à des températures de 80 à 200, plus spécialement de 100 à 180 et de préférence de 120 à 170 ° C.

**6.** Procédé selon revendication 5, caractérisé en ce que l'hydrogénation catalytique est réalisée sous une pression de 1,0 à 6,0, plus spécialement de 1,5 à 5,0 et de préférence de 2,0 à 4,0 MPa.

**7.** Procédé selon une ou plusieurs des revendications 1 à 4, caractérisé en ce que, pour la préparation des amines secondaires, on procéde à l'hydrogénation catalytique des nitriles à des températures de 160 à 250, plus spécialement de 170 à 240 et de préférence de 180 à 220°C.

8. Procédé selon revendication 7, caractérisé en ce que l'hydrogénation catalytique est réalisée sous une pression de 0,1 à 5,0, plus spécialement de 0,2 à 3,0, de préférence de 0,3 à 2,0 MPa avec élimination de l'ammoniac libéré.

9. Procédé selon une ou plusieurs des revendications 1 à 8, caractérisé en ce que le catalyseur sur support contenant du nickel est mis en oeuvre en quantité correspondant à 0,05 à 2,0, plus spécialement 0,08 à 1,0 et de préférence 0,1 à 0,5% en poids de nickel par rapport au nitrile.

10. Procédé selon une ou plusieurs des revendications 1 à 9, caractérisé en ce que l'on met en oeuvre des nitriles contenant de 6 à 24 atomes de carbone, plus spécialement des nitriles aliphatiques à chaîne droite et/ou ramifiée contenant de 8 à 22 atomes de carbone, de préférence des nitriles aliphatiques à chaîne droite et/ou ramifiée contenant 10 à 22 atomes de carbone.